(19) Europäisches Patentamt
European Patent Office
Office européen des brevets

(11) **EP 2 730 213 A1**

(12) **EUROPEAN PATENT APPLICATION**

(43) Date of publication:
**14.05.2014 Bulletin 2014/20**

(51) Int Cl.:
*A61B 3/12* (2006.01)    *G02B 26/06* (2006.01)
*G02B 26/08* (2006.01)

(21) Application number: **13190788.3**

(22) Date of filing: **30.10.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **07.11.2012  JP 2012245863**

(71) Applicant: **Canon Kabushiki Kaisha**
**Tokyo 146-8501 (JP)**

(72) Inventors:
• **Shimada, Yasuhiro**
  **Tokyo 146-8501 (JP)**
• **Ozaki, Hiroyuki**
  **Tokyo 146-8501 (JP)**
• **TAMAMORI, Kenji**
  **Tokyo 146-8501 (JP)**

(74) Representative: **TBK**
**Bavariaring 4-6**
**80336 München (DE)**

(54) **Actuator, deformable mirror, adaptive optics system using the deformable mirror, and scanning laser ophthalmoscope using the adaptive optics system**

(57)    Provided is an actuator including a substrate, a movable portion provided so as to be movable with respect to the substrate, at least three elastic bodies for supporting the movable portion to the substrate in a displaceable manner, a movable comb electrode supported by the movable portion and extending in a direction parallel to a surface of the substrate, and a fixed comb electrode supported by the substrate and extending in the direction parallel to the surface of the substrate in which the movable comb electrode and the fixed comb electrode are arranged so as to be alternately engaged with each other with a distance, and each of all of the elastic bodies has a long axis that forms an angle of more than 90° and 180° or less with respect to at least one of the other elastic bodies.

FIG. 1

**Description**

BACKGROUND OF THE INVENTION

Field of the Invention

The present invention relates to an actuator, a deformable mirror using the actuator, an adaptive optics system using the deformable mirror, and a scanning laser ophthalmoscope using the adaptive optics system.

Description of the Related Art

A movable mirror of a type to be displaced by an electrostatic attractive force is expected to be applied to various fields utilizing light. For example, the movable mirror can be utilized as an adaptive optics wavefront correction device to be installed in a fundus inspection apparatus, an astronomical telescope, or the like. As a representative example of such a movable mirror that is displaced by an electrostatic attractive force, there is known a measure of enabling movement by using two parallel plate electrodes, but this parallel plate type has a disadvantage in that the moving amount is small.

In contrast, in recent years, a deformable mirror that uses comb electrodes and can achieve a larger moving amount has been proposed. An example thereof is disclosed in US Patent No. 6,384,952. As illustrated in FIG. 8, in the deformable mirror, a support portion 530 that supports a comb electrode 520 on a movable side and a support portion 570 that supports a comb electrode 510 on a fixed side are respectively located on the drawing sheet on upper and lower sides in a perpendicular direction. The movable comb electrode and the fixed comb electrode are opposed to each other, and are arranged so as to be alternately arrayed with a distance. With this, an electrode overlapping area larger than that in the parallel plate type can be achieved. Therefore, a larger electrostatic attractive force can be generated between the comb electrodes, and thus a moving amount can be increased.

However, in the structure disclosed in US Patent No. 6,384,952, the fixed comb electrode and its support portion are arranged in a moving direction of the movable comb electrode. Therefore, an excessive electrostatic attractive force may act on the movable comb electrode as compared to a restoring force of a spring acting on the electrode. As a result, in some cases, the comb electrode on the movable side collides with the support portion on the fixed side, which corresponds to a phenomenon called pull-in. Therefore, in this structure, it is not easy to obtain a larger moving amount. Further, the comb electrodes are used, and hence when such a component to displace the movable portion in an unnecessary direction other than the direction parallel to the comb surface is large, the pull-in may occur between the movable comb electrode and the fixed comb electrode, or the movable comb electrode and the fixed comb electrode may be brought into contact with each other.

SUMMARY OF THE INVENTION

The present invention has been made in view of the above-mentioned problems, and has an object to provide an actuator including comb electrodes and a deformable mirror using the actuator, which have a structure that does not cause pull-in even during significant displacement in a moving direction, and have a structure capable of preventing occurrence of pull-in due to displacement in an unnecessary direction.

An actuator according to one embodiment of the present invention employs the following configuration. Specifically, the actuator includes: a substrate; a movable portion provided so as to be movable with respect to the substrate; at least three elastic bodies for connecting the movable portion and the substrate; a movable comb electrode including a plurality of comb teeth each having one end supported by the movable portion and extending from the one end supported by the movable portion in a direction parallel to a surface of the substrate; and a fixed comb electrode including a plurality of comb teeth each having one end supported by the substrate and extending from the one end supported by the substrate in the direction parallel to the surface of the substrate, in which the plurality of comb teeth of the movable comb electrode and the plurality of comb teeth of the fixed comb electrode are arranged so as to alternately engage with each other with a distance. Each of all the at least three elastic bodies has a long axis that forms an angle of more than 90° and 180° or less with respect to a long axis of at least one of the other elastic bodies.

Further, a deformable mirror according to one embodiment of the present invention employs the following configuration. Specifically, the deformable mirror includes: at least one actuator described above; and a mirror portion having a reflective surface, in which the mirror portion is connected to the movable portion of the at least one actuator, and the deformable mirror is capable of changing a shape of the mirror portion by operating the at least one actuator.

Further, according to one embodiment of the present invention, there is provided an adaptive optics system including: a deformable mirror arranged on an optical path, the deformable mirror including an actuator and a mirror portion having a reflective surface connected to the actuator; a wavefront sensor for measuring a wavefront of incident light; and an adaptive optics controller for calculating a shape of the reflective surface to obtain a wavefront with no aberration, based on the wavefront measured by the wavefront sensor, in which the actuator includes the actuator described above, and the adaptive optics controller controls the actuator so that the reflective surface of the deformable mirror has the calculated shape of the reflective surface.

Further, according to one embodiment of the present invention, there is provided a scanning laser ophthalmoscope, which irradiates an eye to be inspected with light to obtain a fundus image based on light intensity of reflected light from a fundus of the eye to be inspected, the scanning laser ophthalmoscope including: a light source; a light scanning portion for scanning light emitted from the light source; and a light intensity sensor for receiving the light intensity of the reflected light from the fundus of the eye to be inspected, in which the adaptive optics system described above is arranged on an optical path from the light source to the eye to be inspected.

Further features of the present invention will become apparent from the following description of exemplary embodiments with reference to the attached drawings.

BRIEF DESCRIPTION OF THE DRAWINGS

FIG. 1 is a perspective view illustrating an actuator according to a first embodiment and Example 1 of the present invention.

FIG. 2 is a top view illustrating the actuator according to the first embodiment and Example 1 of the present invention.

FIGS. 3A and 3B are sectional views illustrating an operation of the actuator according to the first embodiment and Example 1 of the present invention.

FIG. 4 is a perspective view illustrating a part of a deformable mirror according to a second embodiment and Example 2 of the present invention.

FIG. 5 is a sectional view illustrating an operation of the deformable mirror according to the second embodiment and Example 2 of the present invention.

FIG. 6A is a top view of an actuator according to the first embodiment and Example 3 of the present invention, and FIG. 6B is a top view of a comparative example.

FIG. 7 is a top view illustrating an actuator according to the first embodiment and Example 4 of the present invention.

FIG. 8 is a view illustrating a deformable mirror of the prior art.

FIG. 9 is a schematic view of an SLO apparatus according to a third embodiment of the present invention.

DESCRIPTION OF THE EMBODIMENTS

In an actuator and a deformable mirror using the actuator of the present invention, a movable portion that is displaced together with a movable comb electrode that is displaced by an electrostatic attractive force with respect to a fixed comb electrode in a direction perpendicular to a surface of a substrate is supported by at least three elastic bodies with respect to the substrate. Further, each of the elastic bodies has a long axis that forms an angle of more than 90° and 180° or less with respect to a long axis of at least one of the other elastic bodies. In a configuration defined by those conditions, the substrate, the movable portion provided so as to be movable with respect to the substrate, the movable and fixed comb electrodes, and the elastic bodies may be appropriately designed in accordance with the specific requirements. In this manner, it is possible to easily realize a structure capable of preventing occurrence of pull-in due to significant displacement in a moving direction or displacement in an unnecessary direction. Specific structures are described later in embodiments and examples, but as a matter of course, the present invention is not limited thereto. For example, such a structure that three elastic bodies whose long axes form angles of 178°, 91°, and 91° support the movable portion to the substrate is possible.

(First Embodiment)

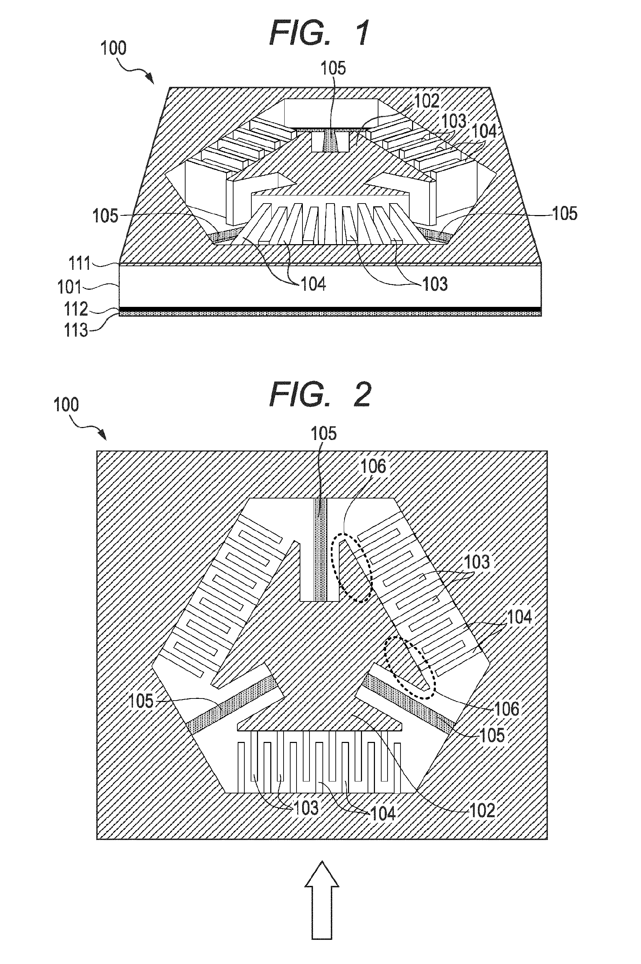

In the following, with reference to FIGS. 1 and 2, an actuator according to the present invention is described. FIG. 1 is a perspective view of the actuator of this embodiment, and FIG. 2 is a top view of the actuator of this embodiment. An actuator 100 is formed by processing a plate-like substrate 101. A movable portion 102 is supported to the substrate 101 by at least three (three in this case) elastic bodies 105 so as to be displaceable in a direction perpendicular to the surface of the substrate. The elastic bodies 105 are each formed of a plate spring, a rod-like spring, or the like, which is capable of being warped at least in the direction perpendicular to the surface of the substrate 101. A movable comb electrode 103 includes a plurality of comb teeth each having one end supported by the movable portion 102. Each of the comb teeth extends in a direction parallel to the surface of the substrate from the one end supported by the movable portion 102. A fixed comb electrode 104 includes a plurality of comb teeth each having one end supported by the substrate 101. Each of the comb teeth extends in the direction parallel to the surface of the substrate from the one end supported by the substrate 101. The movable comb electrode 103 and the fixed comb electrode 104 are arranged so as to be opposed to each other, and the comb teeth thereof are arranged so as to be alternately arrayed with a distance. The opposed comb surfaces extend in parallel to a moving direction of the movable portion 102.

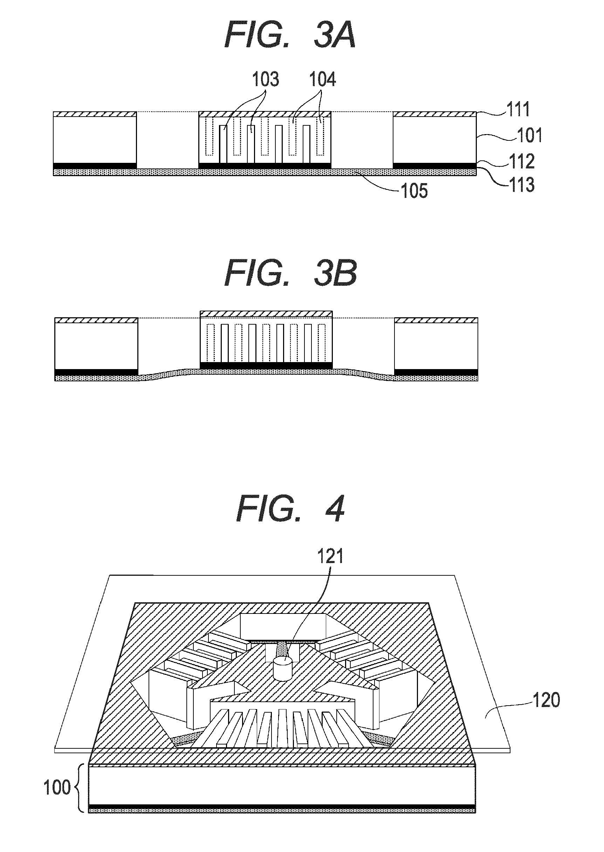

FIGS. 3A and 3B illustrate an operation of the actuator in a state viewed from the direction on an arrow of FIG. 2. In FIGS. 3A and 3B, a front part of the substrate 101 is omitted so that the operation of the actuator can be easily recognized visually. As illustrated in FIG. 3A, in an initial position when no potential difference is applied between the comb electrodes, a level difference is formed between the movable comb electrode 103 and the fixed comb electrode 104 in a direction perpendicular to the surface of the substrate. That is, the comb electrodes have non-overlapping parts in the direction perpendicular to the surface of the substrate. This is because the present invention employs the comb electrodes of a type (variable overlapping type) that utilizes a phenomenon that a force acts and displacement occurs in an overlapping direction when the comb electrodes are attracted to each other by an electrostatic attractive force.

In this phenomenon, no further displacement occurs when the comb electrodes are entirely overlapped with each other, and hence it is required that, in the initial position, the overlapping part be reduced to increase the overlapping part when a voltage is applied. The movable comb electrode 103 and the fixed comb electrode 104 are electrically insulated from each other. When a voltage is applied between the movable comb electrode 103 and the fixed comb electrode 104, while maintaining the distance between the movable comb electrode 103 and the fixed comb electrode 104, the movable portion 102 is displaced in the direction perpendicular to the surface of the substrate. An electrostatic attractive force $Fz$ in a z direction (direction perpendicular to the surface of the substrate), which acts when a potential difference is applied between the movable comb electrode and the fixed comb electrode, may be represented by the following formula (1) :

$$Fz = [(\varepsilon_0 \cdot N \cdot h)/(2g)] \cdot (Vm - Vf)^2 \qquad (1),$$

where $\varepsilon_0$ represents a dielectric constant of vacuum, N represents the number of gaps between the comb electrodes, h represents an overlapping length between the movable comb electrode and the fixed comb electrode, Vm represents a potential of the movable comb electrode, Vf represents a potential of the fixed comb electrode, and g represents a width of the gap between the comb electrodes.

First, in the initial position of FIG. 3A, an electrostatic attractive force is generated when a potential difference is applied between the movable comb electrode 103 and the fixed comb electrode 104, and thus the electrodes are attracted to each other. In this manner, the movable comb electrode 103 and the fixed comb electrode 104 are attracted to each other, but in a direction in which the comb surfaces are opposed to each other, a substantially uniform electrostatic attractive force is applied on left and right sides, and hence displacement occurs in the direction perpendicular to the surface of the substrate. Then, as illustrated in FIG. 3B, the movable comb electrode 103 stops at a position at which the restoring force of the elastic bodies 105 and the electrostatic attractive force that causes displacement of the movable portion 102 are balanced. Subsequently, when the potential difference between the movable comb electrode 103 and the fixed comb electrode 104 is set to 0, the movable comb electrode 103 returns to the initial position by the restoring force of the elastic bodies 105. As illustrated in FIGS. 3A and 3B, even when the electrostatic attractive force is generated to cause attraction between the comb electrodes, neither of the comb electrodes collides with members connected to the other comb electrode.

In the structure disclosed in US Patent No. 6,384,952 described above, the comb electrodes and the support portions are arranged in the direction perpendicular to the surface of the substrate, which is the moving direction of the movable comb electrode. Therefore, when the movable comb electrode is displaced, an electrostatic attractive force may be generated between a leading end surface of the comb electrode and the support portion surface to cause pull-in when an excessive electrostatic attractive force is generated as compared to the restoring force of the spring, and thus collision may occur between the comb electrode and the support portion. However, according to the structure of this embodiment, the support portion is not arranged in the direction perpendicular to the surface of the substrate, which is the moving direction of the movable comb electrode, and hence pull-in does not occur. In other words, with the structure of this embodiment, even when the electrostatic attractive force acts, both of the comb electrodes may pass each other without collision. Therefore, pull-in does not occur, and short-circuit of the electrodes does not occur as well.

This embodiment further includes a structure for suppressing unnecessary displacement other than the displacement in the direction perpendicular to the surface of the substrate. The unnecessary displacement other than the displacement in the direction perpendicular to the surface of the substrate includes two components, that is, translation in in-plane two directions parallel to the surface of the substrate, and rotation in a plane parallel to the substrate and rotation about an axis parallel to the substrate. Considering one set of the movable comb electrode and the fixed comb electrode that engage with each other in an arbitrary orientation, the unnecessary direction that particularly causes a problem is components of translation in a direction perpendicular to opposing surfaces of the comb teeth forming the comb electrodes, and rotation other than rotating about an axis perpendicular to the opposing surfaces of the comb teeth forming the comb electrodes. When displacement in those directions increases, there arises a problem in that pull-in is caused between the movable comb electrode and the fixed comb electrode, or in that the movable comb electrode and the fixed comb electrode are brought into contact with each other.

When the directions of long axes of the plurality of elastic bodies supporting the movable portion are equal to each other, the spring constant of rotation about the long axis is reduced. In this case, rotation about the long axis of the elastic body may easily occur, which corresponds to the unnecessary direction. There is a limit to reduce the thickness of the plate-like elastic body for drive at low voltage. However, when the width of the elastic body is reduced, the spring constant of an unnecessary mode such as torsion is also reduced. Further, when all of the elastic bodies are concentrated on one side of the movable portion, the whole can be regarded as a single cantilever, and hence the warpage of the cantilever may easily occur. This warpage contains a displacement component in a direction other than the direction perpendicular to the surface of the substrate, that is, the unnecessary direction. In view of the above, in the present invention, the movable portion is supported by at least three elastic bodies, and each of all the elastic bodies has a long axis that forms an angle of more than 90° and 180° or less with respect to a long axis of at least one of the other elastic bodies. In this case, the long axis of the elastic body may be considered as a line connecting the center of a fixed end of the elastic body on the substrate side and the center of a fixed end thereof on the movable portion side.

Further, this embodiment may include another structure for reducing displacement of the movable portion in the unnecessary direction. When the size of the movable portion is small, displacement in the unnecessary rotation direction easily occurs. However, on the other hand, when the size of the movable portion is increased, the weight increases, and hence the natural frequency reduces, which causes a problem of reduction in responsiveness. That is, there is a trade-off relationship between suppression of displacement in the unnecessary direction and the responsiveness. In this embodiment, in order to suppress the displacement in the unnecessary rotation direction while limiting the weight of the movable portion to a minimum necessary level, the movable portion is provided with an extending portion that extends from the main body portion, and the end portion of the elastic body is connected to the extending portion. With this, as compared to a case where the same numbers of elastic bodies are connected without the extending portion, the distance between the end portions of the plurality of elastic bodies can be increased, and hence displacement in the unnecessary rotation direction can be suppressed.

Further, this embodiment may include a structure for realizing low voltage drive and large stroke by a small-sized actuator. In order to realize large stroke by low voltage drive, firstly, the number of comb teeth of each of the movable comb electrode and the fixed comb electrode may be increased. When the dimension of a single comb electrode is the same, a larger electrostatic attractive force is generated and a larger displacement is enabled as the number of comb teeth of each of the movable comb electrode and the fixed comb electrode becomes large. Secondly, the spring constant of the elastic body in the moving direction may be small. When the spring constant is small, the movable portion may be displaced in a larger amount at the same drive voltage. However, the above-mentioned two items both require a reasonable area, which cannot be easily realized when the actuator is required to be downsized for arrangement in high density. In this embodiment, in order to realize the above-mentioned two items, the extending portion is provided to the movable portion, and the movable comb electrode is formed on the extending portion. With this, the length of the elastic body is secured (and thus the spring constant of the elastic body in the moving direction can be reduced). In addition, the number of comb teeth can be increased.

(Second Embodiment)

A deformable mirror according to a second embodiment of the present invention, which uses the actuator of the first embodiment, is described.

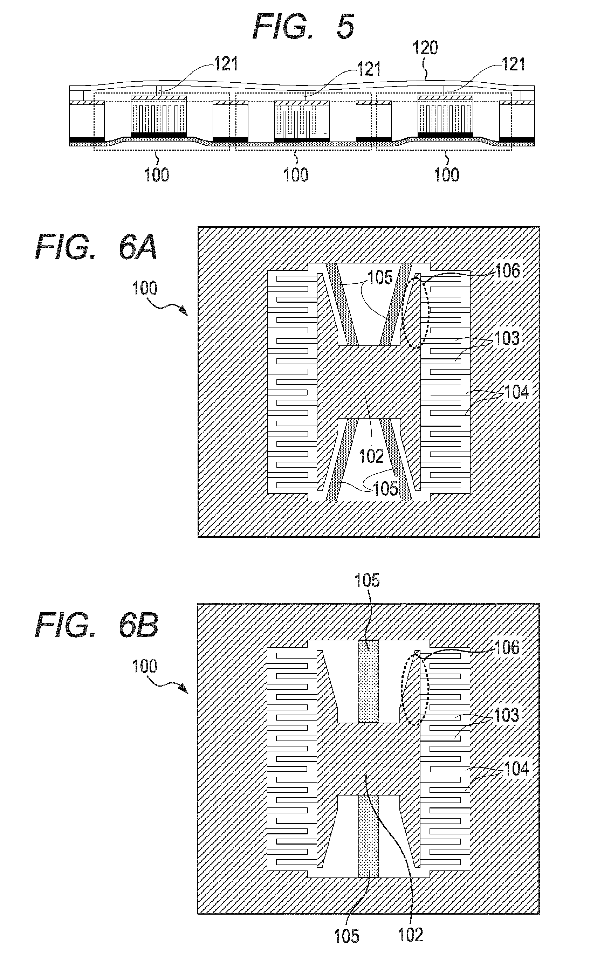

FIGS. 4 and 5 illustrate one mode of the deformable mirror according to this embodiment. FIG. 4 is a perspective view illustrating a part of the deformable mirror, and FIG. 5 is a view illustrating an operation of the deformable mirror. In the deformable mirror, a plurality of the actuators 100 according to the first embodiment are two-dimensionally arranged on the substrate 101, and each of the actuators 100 is connected to a single mirror portion 120 via a connection portion 121.

The structure and the operation principle of each of the actuators 100 are similar to those illustrated in FIGS. 3A and 3B. The deformable mirror of this embodiment can obtain a desired shape by individually displacing the movable portion 102 by individually applying a potential difference between the comb electrodes of each of the actuators 100. With this, the optical path length of light reflected at a desired position of a reflective surface of the mirror portion 120 can be changed, and hence the deformable mirror can be used as a wavefront correction device.

FIGS. 4 and 5 illustrate a type including the single mirror portion 120 connected to the plurality of actuators 100, but a mode in which a mirror portion having a reflective surface is connected to each of the plurality of actuators 100 one by one via the connection portion 121 is also possible.

According to the present invention, it is possible to provide the actuator including the comb electrodes and the deformable mirror using the actuator, which have a structure that does not cause pull-in even during significant displacement in the moving direction, and have a structure capable of preventing occurrence of pull-in due to the displacement in the unnecessary direction.

(Third Embodiment)

An adaptive optics system that uses the deformable mirror described in the second embodiment as a wavefront correction device that compensates for an optical aberration is described with a scanning laser ophthalmoscope (hereinafter described as "SLO apparatus") as an example. The SLO apparatus is an apparatus that irradiates a fundus with light so as to enable observation of a photoreceptor, a retinal nerve fiber layer, hemodynamics, or the like.

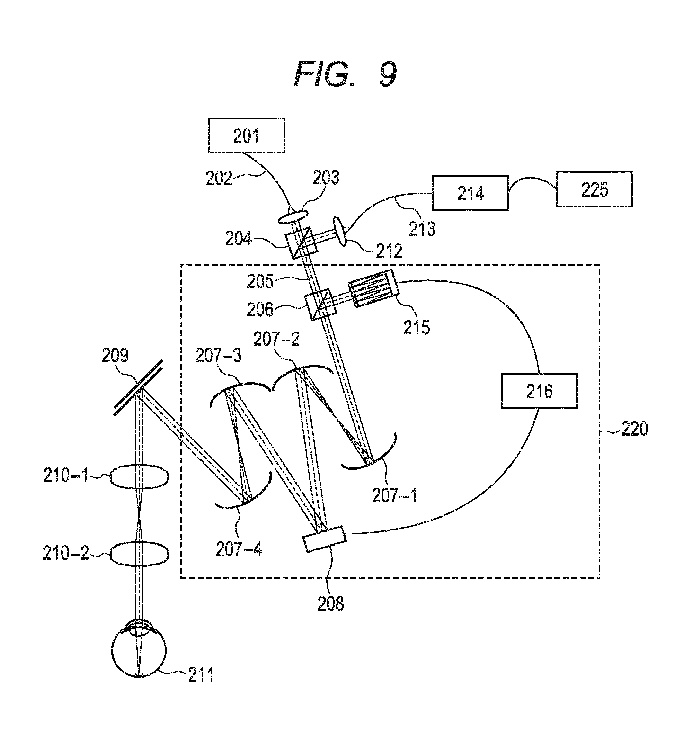

FIG. 9 illustrates a schematic configuration of the SLO apparatus of this embodiment.

Light emitted from a light source 201 travels through a single-mode optical fiber 202 and passes through a collimator 203 to become a collimated light beam. The collimated light beam is transmitted through a beam splitter 204, which serves as light splitting means, as measurement light 205 to be guided to an adaptive optics system 220. The wavelength of the light source 201 is not particularly limited, but particularly for fundus imaging, the wavelength of about 800 nm to 1,500 nm is suitably used for preventing dazzling of a subject and for maintaining the resolution.

The adaptive optics system 220 includes a beam splitter 206 serving as light splitting means, a wavefront sensor (aberration measuring means) 215, a deformable mirror (wavefront correction device) 208, and reflective mirrors 207-1 to 207-4 for guiding the light to those members. The respective reflective mirrors 207 are placed so that at least the pupil of the eye to be inspected, the wavefront sensor 215, and the deformable mirror 208 have an optically conjugate relationship.

The light that has passed through the adaptive optics system 220 is scanned by a light scanning portion 209 one-dimensionally or two-dimensionally. The measurement light scanned by the light scanning portion 209 is radiated to an eye 211 to be inspected through eyepiece lenses 210-1 and 210-2. By adjusting the positions of the eyepiece lenses 210-1 and 210-2, optimum irradiation can be performed in accordance with the visibility of the eye 211 to be inspected. In this case, a lens is used in the eyepiece part, but a spherical mirror or the like may be used instead.

The measurement light radiated to the eye 211 to be inspected is reflected or scattered by a fundus (retina). The light reflected or scattered at the fundus of the eye 211 to be inspected travels, in an opposite direction, a passage similar to that during entrance, and is partially reflected by the beam splitter 206 to enter the wavefront sensor 215. Thus, the wavefront of the light beam is used for measurement. As the wavefront sensor 215, a known Shack-Hartmann sensor can be used.

The reflected and scattered light that has transmitted through the beam splitter 206 is partially reflected by the beam splitter 204 to be guided to a light intensity sensor 214 through a collimator 212 and an optical fiber 213. Light that has entered the light intensity sensor 214 is converted into an electrical signal to be processed into a fundus image by image processing means 225.

The wavefront sensor 215 is connected to an adaptive optics controller 216 to transmit the wavefront of the received light beam to the adaptive optics controller 216. The adaptive optics controller 216 is connected to the deformable mirror 208, which is deformed into a shape instructed by the adaptive optics controller 216.

The adaptive optics controller 216 calculates, based on the wavefront acquired from the wavefront sensor 215, a mirror shape that enables correction into a wavefront with no aberration. Then, in order to reproduce the shape in the deformable mirror 208, a necessary application voltage difference for each of the comb electrodes is calculated and sent to the deformable mirror 208. In the deformable mirror 208, a potential difference sent from the adaptive optics controller 216 is applied between the movable comb electrode and the fixed comb electrode, to thereby deform the mirror surface into a predetermined shape.

The measurement of the wavefront by the wavefront sensor 215, transmission of the wavefront to the adaptive optics controller 216, and instruction by the adaptive optics controller 216 to the deformable mirror for correction of the aberration as described above are repeatedly processed to be feed-back controlled to constantly obtain an optimum wavefront.

When the adaptive optics system according to this embodiment is used, because pull-in does not occur even when the deformable mirror is significantly displaced in the moving direction, the aberration can be compensated for over a wide range. Further, compensation is possible in fast reaction to the instruction from the adaptive optics controller 216. Therefore, the SLO apparatus using the adaptive optics system according to the present invention can appropriately compensate for the aberration generated in the eye to be inspected, and hence high resolution imaging becomes possible.

In the above-mentioned embodiments, various modifications and corrections can be made without departing from the gist of the present invention.

In the following, further specific examples are described.

(Example 1)

Example 1 describes one mode of the actuator according to the present invention. The actuator according to Example 1 is described with reference to FIGS. 1 and 2. FIG. 1 is a perspective view of the actuator according to Example 1, and FIG. 2 is a top view of the actuator according to Example 1. The actuator 100 is formed by processing the substrate 101 made of silicon. The movable portion 102 is supported to the substrate 101 by the three elastic bodies 105. The elastic body 105 is formed by processing an elastic body layer 113 made of silicon, which is formed on the substrate 101 through intermediation of an insulating layer 112. The three elastic bodies 105 have long axes in the longitudinal direction, which form an angle of 120° therebetween. With the structure of Example 1, as compared to the spring constant of rotation about the long axis in a case where two elastic bodies are used for support, which have overlapped long axis directions and have the same spring constant in the direction perpendicular to the substrate, the spring constant of rotation about the long axis of an arbitrary elastic body of Example 1 is increased to about 50 times. With this, an effect of suppressing the rotation in the unnecessary direction can be obtained.

Between end portions of two adjacent elastic bodies 105 formed on the movable portion 102, the movable comb electrode 103 is formed so as to extend from the movable portion 102 in a direction parallel to the surface of the substrate. The fixed comb electrode 104 extends from the substrate 101 in a direction parallel to the surface of the substrate. The movable comb electrode 103 and the fixed comb electrode 104 are arranged so as to be opposed to each other, and the comb teeth thereof are arranged so as to be alternately arrayed. The movable comb electrode 103 and the fixed comb electrode 104 can be formed by dry etching with use of a mask layer 111 as an etching mask.

In Example 1, the movable portion 102 includes six extending portions 106, and the movable comb electrode 103 is also formed on the extending portion 106. With this, as compared to the case where the length of the elastic body is the same and the extending portion is omitted, the drive voltage for obtaining the same stroke can be reduced to about one-half. The operation of the actuator of Example 1 is the same as that described above with reference to FIGS. 3A and 3B.

(Example 2)

Example 2 describes one mode of the deformable mirror according to the present invention. The deformable mirror according to Example 2 is described with reference to FIGS. 4 and 5. FIG. 4 is a perspective view illustrating a part of the deformable mirror according to Example 2, and FIG. 5 is a view illustrating an operation of the deformable mirror according to Example 2. In the deformable mirror according to Example 2, a plurality of the actuators 100 according to Example 1 are two-dimensionally arranged on the substrate 101. Each of the actuators 100 is connected to the single mirror portion 120 via the connection portion 121.

The structure and the operation principle of each of the actuators 100 are similar to those described in Example 1. In the deformable mirror of Example 2, the potential difference between the comb electrodes of each of the actuators 100 is individually controlled to obtain a desired shape by individually displacing the movable portion 102. With this, the optical path length of light reflected at a desired position of the reflective surface of the mirror portion 120 can be changed, and hence the deformable mirror can be used as a wavefront correction device.

(Example 3)

Example 3 describes one mode of the actuator according to the present invention. FIG. 6A illustrates a top view of the actuator according to Example 3. Components thereof are the same as those of a type of FIG. 1 described in Example 1, and hence detailed description thereof is omitted. The operation of the actuator is the same as that described above with reference to FIGS. 3A and 3B.

The actuator of Example 3 includes four elastic bodies. Each single elastic body 105 has a long axis in the longitudinal direction, which forms angles of 30°, 150°, and 180° with respect to the long axes of the other elastic bodies. Now, comparison is considered between the structure of FIG. 6A and the structure of FIG. 6B in which the movable portion is supported by two plate springs which each have a width that is twice the width of the plate spring of FIG. 6A and form an angle of 180° therebetween. As compared to the structure of FIG. 6B, the spring constant of rotation about an axis perpendicular to the comb surface of FIG. 6A is increased to about 20 times, and thus it is possible to obtain an effect of suppressing the rotation of the movable portion in the unnecessary direction. Further, in a case where a substrate for the elastic body part having a large width as in FIG. 6B is subjected to etching simultaneously when the comb teeth are formed by dry etching, because the local etching area differs, the etching progresses faster in the elastic body part than in the comb teeth part, and hence pattern abnormality may occur in some cases. However, by dividing the elastic body part to reduce the width thereof as in Example 3, it is possible to obtain an effect of avoiding such pattern abnormality.

Example 3 further has a structure in which the movable portion 102 includes four extending portions 106, and the movable comb electrode 103 is also formed on the extending portion 106. With this, as compared to the case where the length of the elastic body is the same and the extending portion is omitted, the drive voltage for obtaining the same stroke can be reduced to about one-third. In Example 3, there is a part in which no movable comb electrode 103 is formed between the adjacent elastic bodies 105, but, as a matter of course, the movable comb electrode 103 may be formed in this part.

(Example 4)

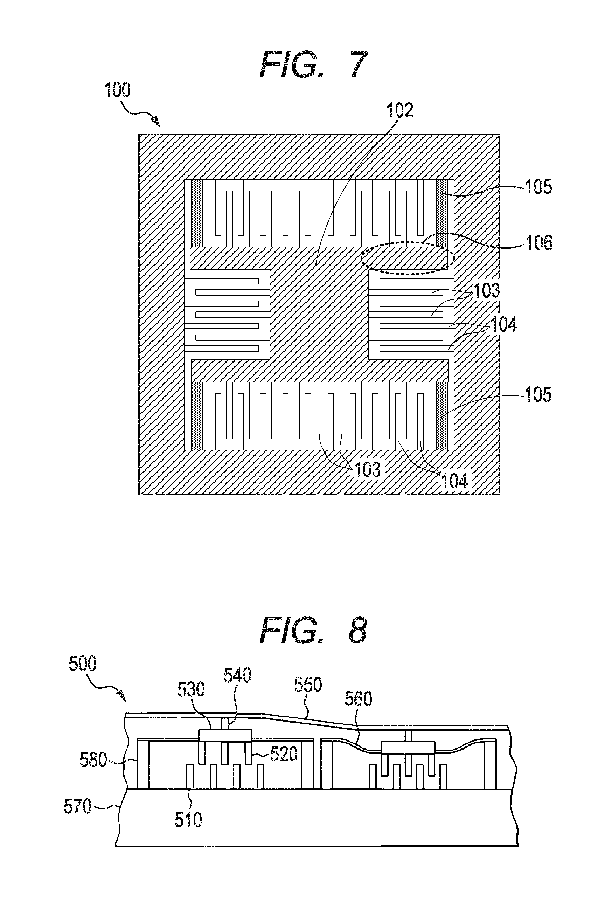

Example 4 describes one mode of the actuator according to the present invention. FIG. 7 illustrates a top view of the actuator according to Example 4. Components thereof are the same as those of a type of FIG. 1 described in Example 1, and hence detailed description thereof is omitted. The operation of the actuator is the same as that described above with reference to FIGS. 3A and 3B.

Example 4 has a structure in which the movable portion 102 includes four extending portions 106 as illustrated in FIG. 7. The movable comb electrode 103 is also formed on the extending portion 106, and the end portions of the four elastic bodies 105 are connected to the respective four extending portions 106. Each single elastic body has a long axis in the longitudinal direction, which forms angles of 0° and 180° with respect to the long axes of the other elastic bodies. Further, a part of the side surface of the extending portion 106 functions as a movable comb electrode by acting with the opposed fixed comb electrode 104. With the structure of Example 4, as compared to the case where the extending portion is omitted, the spring constant obtained when the in-plane direction of the substrate perpendicular to the extending direction of the extending portion 106 is set as a rotational axis is increase to about 5 times, and it is possible to obtain an effect of suppressing the rotation of the movable portion in the unnecessary direction.

Further, the movable comb electrode 103 is also formed on the extending portion 106, and a part of the side surface of the extending portion 106 is caused to function as the movable comb electrode. In this manner, as compared to the case where the movable comb electrode 103 is not formed on the extending portion 106, the drive voltage for obtaining the same stroke can be reduced to about one-half. In Example 4, the longitudinal directions of the elastic bodies are aligned to 0° and 180°, but the present invention is not limited thereto. For example, the elastic bodies may be arranged so that the adjacent elastic bodies have longitudinal directions that form an angle of 90°.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions. Provided is an actuator including a substrate, a movable portion provided so as to be movable with respect to the substrate, at least three elastic bodies for supporting the movable portion to the substrate in a displaceable manner, a movable comb electrode supported by the movable portion and extending in a direction parallel to a surface of the substrate, and a fixed comb electrode supported by the substrate and extending in the direction parallel to the surface of the substrate in which the movable comb electrode and the fixed comb electrode are arranged so as to be alternately engaged with each other with a distance, and each of all of the elastic bodies has a long axis that forms an angle of more than 90° and 180° or less with respect to at least one of the other elastic bodies.

**Claims**

1.  An actuator comprising:

    a substrate;
    a movable portion provided so as to be movable with respect to the substrate;
    at least three elastic bodies for connecting the movable portion and the substrate;
    a movable comb electrode including a plurality of comb teeth each having one end supported by the movable portion and extending from the one end supported by the movable portion in a direction parallel to a surface of the substrate; and
    a fixed comb electrode including a plurality of comb teeth each having one end supported by the substrate and extending from the one end supported by the substrate in the direction parallel to the surface of the substrate,
    wherein the plurality of comb teeth of the movable comb electrode and the plurality of comb teeth of the fixed comb electrode are arranged so as to alternately engage with each other with a distance, and
    wherein each of all the at least three elastic bodies has a long axis that forms an angle of more than 90° and 180° or less with respect to a long axis of at least one of the other elastic bodies.

2.  The actuator according to claim 1,
    wherein the movable portion comprises an extending portion, and
    wherein at least a part of the at least three elastic bodies is connected to the extending portion.

**3.** The actuator according to claim 1,
wherein the movable portion comprises an extending portion, and
wherein at least a part of the plurality of comb teeth of the movable comb electrode is supported by the extending portion.

**4.** A deformable mirror comprising:

at least one actuator according to any one of claims 1 to 3; and
a mirror portion having a reflective surface,

the mirror portion being connected to the movable portion of the at least one actuator,

the deformable mirror being capable of changing a shape of the mirror portion by operating the at least one actuator.

**5.** An adaptive optics system, which compensates for an aberration generated on an optical path, the adaptive optics system comprising:

a deformable mirror arranged on the optical path, the deformable mirror including an actuator and a mirror portion having a reflective surface connected to the actuator;
a wavefront sensor for measuring a wavefront of incident light; and
an adaptive optics controller for calculating a shape of the reflective surface to obtain a wavefront with no aberration, based on the wavefront measured by the wavefront sensor,
wherein the actuator comprises the actuator according to any one of claims 1 to 3, and
wherein the adaptive optics controller controls the actuator so that the reflective surface of the deformable mirror has a calculated shape of the reflective surface.

**6.** A scanning laser ophthalmoscope, which irradiates an eye to be inspected with light to obtain a fundus image based on light intensity of reflected light from a fundus of the eye to be inspected, the scanning laser ophthalmoscope comprising:

a light source;
a light scanning portion for scanning light emitted from the light source; and
a light intensity sensor for receiving the light intensity of the reflected light from the fundus of the eye to be inspected,
wherein the adaptive optics system according to claim 5 is arranged on an optical path from the light source to the eye to be inspected.

# FIG. 1

# FIG. 2

## FIG. 3A

## FIG. 3B

## FIG. 4

## FIG. 5

## FIG. 6A

## FIG. 6B

## FIG. 7

## FIG. 8

# FIG. 9

Europäisches
Patentamt

European
Patent Office

Office européen
des brevets

## EUROPEAN SEARCH REPORT

Application Number

EP 13 19 0788

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | W. NOELL ET AL: "<title>Compact large-stroke piston tip-tilt actuator and mirror</title>", PROCEEDINGS OF SPIE, vol. 6467, 6 February 2007 (2007-02-06), pages 64670Q-64670Q-7, XP055090805, ISSN: 0277-786X, DOI: 10.1117/12.698937 | 1-5 | INV. A61B3/12 G02B26/06 G02B26/08 |
| Y | * pages 64670q-1 - pages 64670q-5; figures 1,2,4,5 * | 6 | |
| Y | WO 2010/119915 A2 (CANON KK [JP]; SAITO KENICHI [JP]) 21 October 2010 (2010-10-21) * pages 1,7 - page 11; figure 2A * | 6 | |
| A,D | US 6 384 952 B1 (CLARK RODNEY L [US] ET AL) 7 May 2002 (2002-05-07) * columns 2,5 - column 7; figures 1,5 * | 1-6 | |
| | | | TECHNICAL FIELDS SEARCHED (IPC) |
| | | | A61B G02B B81B |

The present search report has been drawn up for all claims

| Place of search | Date of completion of the search | Examiner |
|---|---|---|
| Munich | 29 January 2014 | Feeney, Orla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                EP 13 19 0788

This annex lists the patent family members relating to the patent documents cited in the above-mentioned European search report.
The members are as contained in the European Patent Office EDP file on
The European Patent Office is in no way liable for these particulars which are merely given for the purpose of information.

29-01-2014

| Patent document cited in search report | | Publication date | Patent family member(s) | | Publication date |
|---|---|---|---|---|---|
| WO 2010119915 | A2 | 21-10-2010 | CN | 102395912 A | 28-03-2012 |
| | | | EP | 2419001 A2 | 22-02-2012 |
| | | | JP | 2010246653 A | 04-11-2010 |
| | | | KR | 20120022890 A | 12-03-2012 |
| | | | RU | 2011146074 A | 20-05-2013 |
| | | | US | 2012002165 A1 | 05-01-2012 |
| | | | WO | 2010119915 A2 | 21-10-2010 |
| US 6384952 | B1 | 07-05-2002 | US | 6384952 B1 | 07-05-2002 |
| | | | US | 2002109894 A1 | 15-08-2002 |

EPO FORM P0459

For more details about this annex : see Official Journal of the European Patent Office, No. 12/82

**EP 2 730 213 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6384952 B